# EUROPÄISCHE PATENTANMELDUNG

(11) **EP 1 347 283 A2**
(43) Veröffentlichungstag der Anmeldung: **24.09.2003**
(21) Anmeldenummer: 03004424.2
(22) Anmeldetag: 27.02.2003
(51) Int. Cl.: G01N 19/08, G01N 3/20, G01N 33/34

(54) **Vorrichtung und Verfahren zur Bestimmung inhomogenitäten in Materialbahnen**

(30) Priorität: 19.03.2002 DE 10212057
(71) Anmelder: tesa AG, 20253 Hamburg (DE)
(72) Erfinder: Hoelger, Christof-Gottfried, Dr., 77749 Hohberg (DE); Becht, Oliver, 79117 Freiburg (DE)

(57) **Zusammenfassung**

Die Erfindung betrifft eine Vorrichtung und ein Verfahren zur Bestimmung von Inhomogenitäten in Materialbahnen. Aufgabe der Erfindung ist es, eine Vorrichtung und ein Verfahren zur Bestimmung von Inhomogenitäten in Materialbahnen bereitzustellen, mit denen wirksam das Vorhandensein und der Grad der Inhomogenität bestimmt werden kann. Erfindungsgemäß wird diese Aufgabe dadurch gelöst, dass über eine Einrichtung (10) eine Materialbahn (30) mit einem Druck beaufschlagt wird und ein der Materialbahn (30) zugeordnetes Meßsystem (20) die Auslenkung der Materialbahn (30) auf Grund der Beaufschlagung mit dem Druck erfasst.

## Beschreibung

Die Erfindung betrifft eine Vorrichtung und ein Verfahren zur Bestimmung von Inhomogenitäten in Materialbahnen. Die Vorrichtung und das Verfahren sind insbesondere dazu geeignet, Unregelmäßigkeiten bei Papier- oder Folienbahnen zu erkennen, um eine Längendifferenz einzelner Sektoren innerhalb der Longitudinalebene zu ermitteln.

Im Bereich der Papierverarbeitung bzw. Folienverarbeitung treten Verarbeitungsprobleme auf, sofern das Papier bzw. die Folie unregelmäßige Strukturen aufweist, da dadurch die Verarbeitung bzw. Anwendung der Papier- oder Folienbahnen erschwert oder unmöglich gemacht wird. In diesem Zusammenhang wird von einem so genannten Trägerverzug gesprochen, bei dem es sich nicht um eine Dickendifferenz oder eine sichtbare Materialveränderung handelt, sondern um eine Längendifferenz innerhalb einzelner Sektoren der Materialbahn innerhalb der Longitudinalebene.

Solche Inhomogenitäten zu erkennen und den Grad der Unregelmäßigkeit zu bestimmen, stößt bislang auf große Schwierigkeiten.

Aufgabe der vorliegenden Erfindung ist es, ein Verfahren und eine Vorrichtung zur Bestimmung von Inhomogenitäten in Materialbahnen bereitzustellen, mit denen wirksam das Vorhandensein und der Grad der Inhomogenität bestimmt werden kann.

Erfindungsgemäß wird diese Aufgabe durch eine Vorrichtung mit den Merkmalen 1 sowie durch ein Verfahren mit den Merkmalen 10 gelöst.

Durch das Beaufschlagen der Materialbahn mit einem Medienstrom, vorzugsweise Luft, oder mit einem Unterdruck und der Erfassung der Auslenkung der Materialbahn auf Grund der Beaufschlagung mit dem Medium oder dem Unterdruck ist es möglich, zuverlässig Inhomogenitäten in untersuchten Materialbahnen zu ermitteln. Neben einer getrennten Anordnung zur Beaufschlagung der Materialbahn mit einem Medienstrom oder einem Unterdruck können diese Maßnahmen auch kombiniert angewendet werden, dass heißt, auf einer Seite wird ein Luft- oder Flüssigkeitsstrom auf die Materialbahn gelenkt und auf der anderen Seite ein Unterdruck angelegt. Auf diese Weise kann die Wirkung der Einrichtung bzw. der Grad der Auslenkung der Materialbahn erhöht werden. Das der Materialbahn zugeordnete Meßsystem erfasst die Auslenkung, und auf Grund der Schwankung bzw. Veränderung der Auslenkung kann die Inhomogenität der Materialbahn bestimmt werden.

Die Beaufschlagungseinrichtung für den Medienstrom oder den Unterdruck sowie das Meßsystem sind vorteilhafterweise relativ zu der Materialbahn beweglich angeordnet, um unterschiedliche Bereiche untersuchen zu können, so dass ein Beaufschlagen der Materialbahn mit einem Medienstrom bzw. Unterdruck nicht über die gesamte Ausdehnung der Materialbahn erfolgen muss.

Um eine genaue Zuordnung der örtlich begrenzten Auslenkung der Materialbahn zu erhalten, ist es vorgesehen, dass sowohl die Beaufschlagungseinrichtung als auch das Meßsystem verschieblich auf einer Führungseinrichtung, insbesondere einer Schiene, angeordnet sind, wobei vorteilhafterweise die Materialbahn unterhalb der Schiene vorbeigeführt wird, um so die gesamte Materialbahn untersuchen zu können.

Die Beaufschlagungseinrichtung, vorzugsweise eine Luftdüse oder eine Absauganlage, ist aus Gründen der einfachen Handhabbarkeit mit dem Meßsystem zur Erfassung der Auslenkung zu einer Prüfeinheit zusammengefasst, so dass diese zusammen ein integriertes Prüfelement bilden.

Der Druck des Mediums bzw. der angelegte Unterdruck ist einstellbar, so dass unterschiedliche Materialien, die unterschiedliche Festigkeiten aufweisen, auf einer Vorrichtung untersucht werden können.

Um ein möglichst präzises und verschleissfreies Erfassen der Auslenkung und damit der Inhomogenität bzw. des Trägerverzuges durchführen zu können, ist das Meßsystem vorteilhafterweise als ein berührungslos arbeitendes Abstandsmeßsystem ausgebildet, so dass keine Taster oder dergleichen auf der Materialbahn angeordnet werden müssen bzw. auf dieser entlang rollen. Um eine möglichst präzise Messung durchzuführen, ist das Meßsystem vorteilhafterweise als eine Laser-Abstandsmesseinrichtung ausgebildet.

Zur gezielten Untersuchung, beispielsweise um statistisch gesicherte Stichprobenmessungen durchzuführen, ist das Meßsystem und die Beaufschlagungseinrichtung bzw. die Prüfeinheit angetrieben verfahrbar ausgebildet, so dass auf einfache Art und Weise ein Prüfprogramm abgefahren werden kann. Wenn die Erfassung der Auslenkung der Materialbahn während des Vorbeiführens an dem Abstandssmeßsystem durchgeführt wird, kann eine Bestimmung der Inhomogenität bzw. des Trägerverzuges während des Herstell- oder Verarbeitungsvorganges (inline) erfolgen, so dass auf Grund der Messergebnisse der Weiterverarbeitungsprozess bzw. der Herstellungsprozess entsprechend angepasst werden kann.

Vorteilhafterweise erfolgt eine Verfahrbewegung des Abstandsmeßsystems und damit auch der Einrichtung zur Beaufschlagung mit einem Medium oder mit Unterdruck quer zur Richtung des Vorbeiführens der Materialbahn, so dass die gesamte Fläche der Materialbahn überstrichen werden kann. Die Messung erfolgt vorteilhafterweise in unmittelbarer Nähe der Beaufschlagung mit dem Medium oder mit dem Unterdruck, um die Kraftanleitung auf die Materialbahn auf ein Minimum beschränken zu können.

Um Optimierugns- bzw. Korrekturmaßnahmen in Bezug auf den Trägerverzug oder die Materialbahn möglichst unmittelbar durchführen zu können, ist es vorgesehen, dass das Entfernungsmeßsystem ein elektrisches Signal ausgibt, das an der rechten Einheit zur Auswertung bzw. Darstellung übermittelt wird und das auf Grund der übermittelten Signale der Ort und der Grad an Inhomogenität der Materialbahn bestimmt wird. Auf diese Weise ist neben einer Bestimmung der Qualität der Materialbahnen, beispielsweise aus Papier, Folie oder Gewebe, die Gewährleistung einer gleichbleibenden Güte der Materialbahn möglich. Ebenfalls können Grenzwerte bezüglich der Auslenkung bzw. des Trägerverzuges festgelegt werden, bis zu denen eine Weiterverarbeitung des Materials zulässig ist; wird ein Grenzwert überschritten, können geeignete Maßnahmen im Herstellungsprozess und oder im weiteren Verlauf der Be- bzw. Verarbeitung getroffen werden.

Nachfolgend wird anhand der Figuren ein Ausführungsbeispiel näher erläutert. Es zeigen:
- Figur 1: eine Draufsicht auf eine erfindungsgemäße Vorrichtung; und
- Figur 2: eine Seitenansicht der Vorrichtung gemäß der Figur 1.

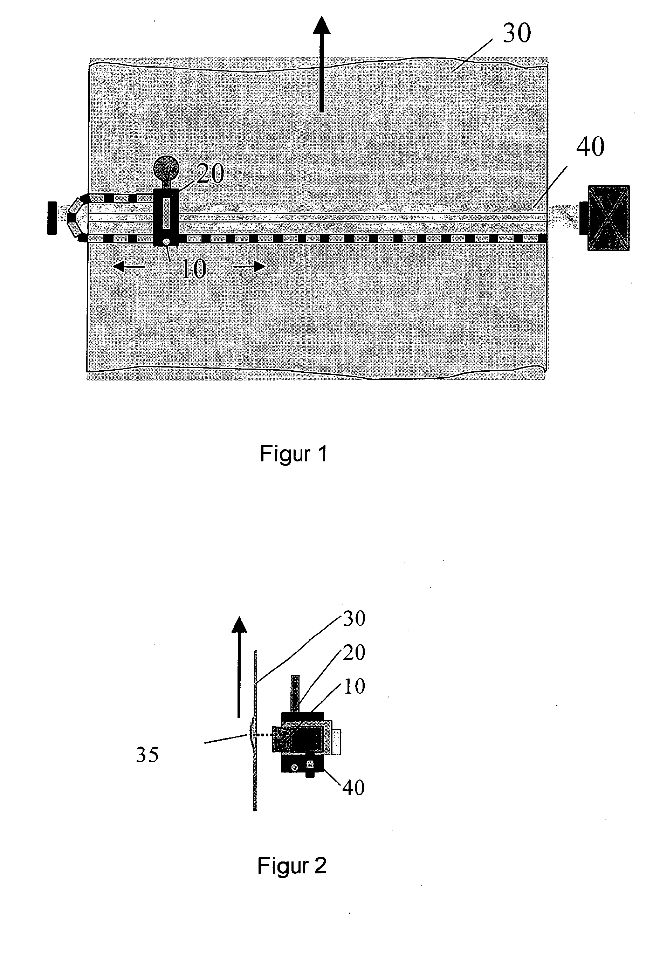

Figur 1 zeigt in Draufsicht mit einer auf einer Führungsschiene 40 angeordneten Einrichtung 10 zum einseitigen Beaufschlagen einer unter Führungsschiene 40 vorbeigeführten Materialbahn 30 sowie einem der Materialbahn 30 zugeordneten Meßsystem 20, das den Abstand der Materialbahn 30 von dem Meßsystem 20 bzw. einem Referenzpunkt erfasst. Die Materialbahn 30 wird unter der Führungsschiene 40 und damit unter der Beaufschlagungseinrichtung 10 und dem Meßsystem 20 in Pfeilrichtung hindurch geführt.

Die Beaufschlagungseinrichtung 10 sowie das Meßsystem 20 sind zu einer Prüfeinheit zusammengefasst und entlang der Führungsschiene 40 quer zur Vorschubrichtung der Materialbahn 30 beweglich, wie durch die Pfeile angedeutet ist. Die Prüfeinheit kann die gesamte Breite der Materialbahn 30 überstreichen, so dass eine vollständige Prüfung hinsichtlich vorhandener Inhomogenitäten vorgenommen werden kann. Die Prüfeinheit ist antreibbar und kann automatisch verfahren werden.

Figur 2 zeigt die Vorrichtung in einer Seitenansicht, bei der die Vorschubbewegung der Materialbahn 30 durch den Pfeil bezeichnet ist. In einem Abstand zu der Materialbahn 30 ist die Führungsschiene 40 angeordnet, auf der senkrecht zu der Darstellungsebene beweglich das Meßsystem 20 in Gestalt eines Laser-Abstandsmessgerätes sowie die Beaufschlagungseinrichtung 10 in Gestalt einer Luftdüse angeordnet ist. Versorgungsleitungen sind in einem Kabelschlepp angeordnet, so dass eine Querbewegung der Prüfeinheit einfach durchgeführt werden kann. In der Figur 2 ist eine Aussackung 35 oder Auslenkung der Materialbahn 30 auf Grund des auf die Materialbahn 30 gerichteten Luftstromes aus der Luftdüse 10 gezeigt. Die Auslenkung 35 wird über das Laser-Abstandsmeßsystem 20 erfasst und auf Grund der Vergrößerung des Abstandswertes der Materialbahn 30 zu dem Laser-Abstandsmeßsystem 20 wird eine charakteristische Kenngröße bzw. ein Signal für das Maß der Stärke der Inhomogenität bzw. des Trägerverzuges ermittelt. Je größer die Auslenkung 35 bei einem Anblasdruck ist, desto größer ist der Trägerverzug, also die Längendifferenz einzelner Bereiche innerhalb der Longitudinalebene der Materialbahn 30.

Neben einem Luftstrom kann durch die Beaufschlagungseinrichtung 10 auch ein anderes Medium auf die Materialbahn 30 gelenkt werden, wobei zudem der Druck und die Form des Beaufschlagungsstromes frei gewählt werden kann. Alternativ oder ergänzend dazu kann eine Unterdruckeinrichtung vorgesehen sein, die die Materialbahn 30 ansaugt. Korrespondierend wird dann bei entsprechender Anordnung des Meßsystems 20 und der Beaufschlagungseinrichtung 10 bei einer Verringerung des Abstandes der Materialbahn 30 zu dem Meßsystem 20 auf einen erhöhten Trägerverzug geschlossen.

Ebenfalls ist es möglich, das Meßsystem 20 und die Beaufschlagungseinrichtung 10 auf gegenüberliegenden Seiten der Materialbahn 30 anzuordnen, wobei gewährleistet sein sollte, dass die Erfassung der Auslenkung in der Nähe der Beaufschlagung der Materialbahn 30 mit dem Medienstrom bzw. Unterdruck erfolgt. Durch eine gegenüberliegende Anordnung des Meßsystems 20 und der Beaufschlagungseinrichtung 10 kann gewährleistet werden, dass die Messung punktgenau unmittelbar an der Beaufschlagungsstelle stattfindet.

## Patentansprüche

1. Vorrichtung zur Bestimmung von Inhomogenitäten in Materialbahnen, insbesondere in Papier- oder Folienbahnen, **gekennzeichnet durch** eine Einrichtung (10) zur Druckbeaufschlagung der Materialbahn (30) und einem der Materialbahn (30) zugeordneten Meßsystem (20) zur Erfassung der Auslenkung der Materialbahn (30) aufgrund der Druckbeaufschlagung.

2. Vorrichtung nach Anspruch 1, **dadurch gekennzeichnet, dass** die Beaufschlagungseinrichtung (10) und das Meßsystem (20) relativ zu der Materialbahn (30) beweglich angeordnet ist.

3. Vorrichtung nach Anspruch 1 oder 2, **dadurch gekennzeichnet, dass** die Beaufschlagungseinrichtung (10) und das Meßsystem (20) verschieblich auf einer Führungseinrichtung (40), insbesondere einer Schiene, angeordnet sind.

4. Vorrichtung nach einem der voranstehenden Ansprüche, **dadurch gekennzeichnet, dass** die Beaufschlagungseinrichtung (10) aus einer Luftdüse oder einer Absauganlage oder einer mechanischen Druckbeaufschlagungseinrichtung besteht.

5. Vorrichtung nach einem der voranstehenden Ansprüche, **dadurch gekennzeichnet, dass** die Beaufschlagungseinrichtung (10) und das Meßsystem (20) zu einer Prüfeinheit zusammengefaßt sind.

6. Vorrichtung nach einem der voranstehenden Ansprüche, **dadurch gekennzeichnet, dass** der Druck des Mediums oder der Unterdruck einstellbar ist.

7. Vorrichtung nach einem der voranstehenden Ansprüche, **dadurch gekennzeichnet, dass** das Meßsystem (20) ein berührungslos arbeitendes Abstandsmeßsystem ist.

8. Vorrichtung nach einem der voranstehenden Ansprüche, **dadurch gekennzeichnet, dass** das Meßsystem (20) als Laser-Abstandsmeßsystem ausgebildet ist.

9. Vorrichtung nach einem der Ansprüche 2 bis 8, **dadurch gekennzeichnet, dass** das Meßsystem (20) und die Beaufschlagungseinrichtung (10) angetrieben verfahrbar ausgebildet sind.

10. Verfahren zur Bestimmung von Inhomogenitäten in einer Materialbahn (30), **dadurch gekennzeichnet, dass** die Materialbahn (30) mit einem Medium und/oder einem Unterdruck beaufschlagt wird und dass die Auslenkung der Materialbahn (30) im Bereich der Beaufschlagung mit dem Medium oder dem Unterdruck über ein Abstandsmeßsystem (20) erfaßt wird.

11. Verfahren nach Anspruch 10, **dadurch gekennzeichnet, dass** die Erfassung der Auslenkung während eines Vorbeiführens der Materialbahn (30) an dem Abstandsmeßsystem (20) durchgeführt wird.

12. Verfahren nach Anspruch 10 oder 11, **dadurch gekennzeichnet, dass** die Erfassung der Auslenkung berührungslos erfolgt.

13. Verfahren nach einem der Ansprüche 10 bis 12, **dadurch gekennzeichnet, dass** das Abstandsmeßsystem (20) quer zur Richtung des Vorbeiführens der Materialbahn (30) verfahren wird.

14. Verfahren nach einem der Ansprüche 10 bis 13, **dadurch gekennzeichnet, dass** die Beaufschlagung der Materialbahn (30) mit dem Medium oder Unterdruck in unmittelbarer Nähe des Abstandsmeßsystems (20) erfolgt.

15. Verfahren nach einem der Ansprüche 10 bis 14, **dadurch gekennzeichnet, dass** das Abstandsmeßsystem (20) ein elektrisches Signal ausgibt, das einer Rechnereinheit zur Auswertung und/oder Darstellung übermittelt wird.

16. Verfahren nach Anspruch 15, **dadurch gekennzeichnet, dass** anhand der Abstandsmeßsystemsignale der Ort und der Grad der Inhomogenität der Materialbahn (30) bestimmt wird.
